# EUROPEAN PATENT SPECIFICATION

(11) **EP 3 275 404 B1**
(45) Date of publication and mention of the grant of the patent: **20.10.2021**
(21) Application number: 15885936.3
(22) Date of filing: 14.05.2015
(51) Int. Cl.: A61F 2/24

(54) **SAFE VALVE SCAFFOLD AND VALVE REPLACEMENT DEVICE WITH VALVE SCAFFOLD**
SICHERES HERZKLAPPENGERÜST UND KLAPPENERSATZVORRICHTUNG MIT DEM HERZKLAPPENGERÜST
ÉCHAFAUDAGE DE VALVULE DE SÉCURITÉ ET DISPOSITIF DE REMPLACEMENT DE VALVULE AVEC ÉCHAFAUDAGE DE VALVULE

(30) Priority: 26.03.2015 CN 201510136304
(43) Date of publication of application: 31.01.2018
(60) Divisional application: 21195080.3
(73) Proprietor: Venus MedTech (HangZhou), Inc., Hangzhou, Zhejiang 310052 (CN)
(72) Inventor: ZENG, Frank, Hangzhou Zhejiang 310000 (CN); LO, Larry, Hangzhou Zhejiang 310000 (CN); QI, Jess, Hangzhou Zhejiang 310000 (CN)
(74) Representative: KIPA AB
(86) International application number: PCT/CN2015/078944
(87) International publication number: WO 2016/149998

(56) References cited:
- EP-A1- 2 520 249
- WO-A1-2009/033469
- WO-A1-2014/127750
- WO-A1-2014/210124
- WO-A1-2015/065646
- WO-A2-2011/072084
- CN-A- 102 113 921
- CN-A- 103 598 939
- CN-U- 204 581 599
- US-A1- 2010 094 411
- US-A1- 2010 268 332
- US-A1- 2014 222 142
- None

## Description

### FIELD OF THE INVENTION

The present invention relates to the technical field of medical apparatuses, and in particularly to a valve stent and a valve replacement device having the same.

### BACKGROUND

In the prior art, an interventional cardiac valve generally includes a compressible valve stent and a plurality of leaflets, wherein the valve stent has good biological compatibility, and can be positioned at a corresponding cardiac valve location safely, stably, and reliably.

A main body of a valve stent is usually a rhombic unit structure, which is configured to meet the requirement of compressibility. However, the rhombic unit structure may form independent rhombic vertices, and these rhombic vertices existing in isolation are usually sharp. During the use process of the valve stent, there is a risk that the rhombic vertices existing in isolation pierce a sheath.

If the rhombic vertices existing in isolation are gathered at an end portion of the valve stent, the risk of piercing is relatively low. However, in order to be fit for particular structures of different valves of hearts, valve stents used in different positions have different structures.

Taking a pulmonary valve as an example, in order to make the location of the valve at the main pulmonary artery more stable, the valve stent is further provided with a flared section located at a branch location of the main pulmonary artery, that is, the rhombic units structure at the end portion of the valve stent are extended further in an axial direction of the valve stent and expanded in a radial direction of the valve stent, such that this portion is softer and can resiliently match with a blood vessel wall, thereby preventing the blood vessel wall from being stabbed or even pierced. However, the changes of the structure on the end portion may cause some vertices of the rhombic unit structures of the valve stent, which exist in isolation, to be exposed.

For example, a Chinese patent application publication CN102961199A discloses a pulmonary artery valve stent which can prevent displacements. The pulmonary artery valve stent includes a valve suture section, an artificial valve connected with the valve suture section, and a position limiting structure connected with a distal end of the valve suture section. The valve suture section is located in a right ventricular outflow tract or a main body of the pulmonary artery after being released, and a vertex portion of the position limiting structure abuts against an intersecting portion of the main body of the pulmonary artery and a branch of the pulmonary artery after being released, thereby providing an axial limiting function. In this patent document, the valve suture section is formed by a plurality of rhombic structure units; when the distal end of the valve suture section is connected with the position limiting structure, some rhombic vertices of the rhombic structure existing in isolation may remain, and these rhombic vertices existing in isolation are potential safety hazards during the use process.

For another example, a Chinese patent CN101951858B discloses a funnel shaped throttling device. As shown in Fig. 1, the funnel shaped throttling device includes a middle portion having rhombic grid structures, and two end portions of flared shape connected to two ends of the middle portion respectively. The middle rhombic grid structures has a plurality of rhombic vertices 10 existing in isolation, may cause much inconvenience during operation process.

For a further example, document WO2009/033469A1 discloses a typical heart valve stent having a section equipped to receive a heart valve implant and a plurality of proximally disposed anchoring elements,

When a valve stent is compressed inwards a sheath pipe, the rhombic vertices existing in isolation will become spines. The spines are prone to stab the sheath pipe when passing through complex and curved anatomic paths in a human body. In the subsequent process of releasing valve, they may also result in too much resistance and pierce the sheath pipe, and thereby cause the valve to be unable to release successfully; in extreme cases, they may stab or even pierce blood vessel walls, and then cause great harm to patients.

### SUMMARY OF THE INVENTION

The present invention provides a valve stent and a valve replacement device with the valve stent, which eliminates rhombic vertices existing in isolation which are present in non-end portions of the valve stent, preventing spines from appearing after the valve stent is compressed while maintaining the mechanical properties of the original structural, and thereby resolving the problem of sheath damaged caused by the spines.

A valve stent comprises a tubular supporting net frame the supporting net frame having opposing first and second frame ends, and the first frame end of the supporting net frame has end nodes; and a flared section connected to the first frame end of the supporting net frame, wherein the flared section comprises a plurality of curved supporting bars and a plurality of guiding bars, wherein each supporting bar has two supporting bar ends, two adjacent supporting bar ends of two adjacent supporting bars are connected to one end node, and each guiding bar has opposing first and second guiding bar ends, with the first guiding bar ends of two adjacent guiding bars respectively connected to two different end nodes, and the second guiding bar ends of two adjacent second guiding bar ends respectively connected to two different positions of the same supporting bar, and the two adjacent guiding bars do not intersect each other.

The supporting net frame of the present invention refers to a portion of the valve stent that is configured to support a prosthesis valve, it is generally tubular, blood flows inside the tubular structure and interacts with the prosthesis valve in the tubular structure. The supporting net frame is not limited to a cylinder extending with a constant diameter, and end portions of the supporting net frame may expand or reduce radially.

At least one end of the supporting net frame is connected with the flared section. An existing valve stent is generally provided with fixed flared sections at both two ends, and the two flared sections form an inflow portion and an outflow portion respectively; the inflow portion and the outflow portion are differentiated according to blood flowing directions, that is, blood enters the valve stent via the inflow portion, passes through the supporting net frame, and leaves the valve stent via the outflow portion.

The supporting net frame has rhombic structures, and all the end nodes at the supporting net frame, namely all the vertices of the rhombic structures adjacent to the flared section, are connected with the flared section to prevent isolated rhombic vertices from appearing at non-end portions of the valve stent, and thus the phenomenon that spines are formed after the valve stent is compressed into the sheath pipe is eliminated.

An end of the valve stent that will be released from the sheath firstly is a precedent release end, the flared section is located at the precedent release end, avoiding the phenomenon that spines pierce the sheath when the sheath is retracted. Even if spines are formed at a posterior release end of the valve stent, as the orientations of the spines are similar to the retracting direction of the sheath pipe, and thus the possibility to cause the phenomenon that the spines pierce the sheath is low. Therefore, preferably, the flaring portion is located at the precedent release end of the valve stent. In the prior art, the outflow portion is generally the precedent release end, and the outflow portion is namely the flared section.

An outer rim of the flaring portion is formed by a plurality of curved supporting bars, end nodes of the supporting net frame that correspond to the locations of the supporting bars are all connected with the supporting bars. The end nodes intersect the supporting bars, or are connected with the supporting bars tangentially and intersectingly by guiding bars.

All of the end nodes are connected to the supporting bars directly or by the guiding bars, that is, there is no end node existing in isolation at the supporting net frame, and each end node is connected with at least three linear edges; when the whole valve stent is compressed, the end nodes will not become spines.

Two ends of each supporting bar are connected with respective end nodes of the supporting net frame, a middle portion of the supporting bar extends along an axial direction of the supporting net frame, and a portion of the supporting bar near the end nodes is bent outwards to form a flared structure. Each guiding bar gradually deviates from the supporting net frame along an extending path from a corresponding end node to a corresponding supporting bar.

The guiding bars are curved appropriately, and their curvature degrees are adapted for the curved shapes of the supporting bars, such that the guiding bars and the supporting bars are located on the same smooth curved surface.

Preferably, an included angle between a line connecting two ends of the extending path and an axis of the valve stent is in the range of 0-70 degrees. When the included angle between the extending path and the axis of the valve stent is 0 degree, the length of each guiding bar is the shortest; however, since the guiding bars and the supporting bars are curved, the extending path of each guiding bar is generally not parallel to the axis of the valve stent. The guiding bars need to be converged to the supporting bars, in order to be adapted for the shapes of the supporting bars, it is difficult to form a large included angle between the extending path of each guiding bar and the axis of the valve stent.

Preferably, the included angle between a line connecting the two ends of the extending path and the axis of the valve stent is in the range of 20-60 degrees. Further preferably, the included angle between the line connecting the two ends of the extending path and the axis of the valve stent is in the range of 30-45 degrees.

Each guiding bar is connected with a supporting bar that is nearest to the guiding bar, and an intersection angle formed between the guiding bar and the supporting bar at the junction is an acute angle. Using such a structure is easy to maintain the original mechanical performance.

Every four adjacent end nodes are assigned as a group. In each group of end nodes, two ends of a supporting bar are respectively connected with two end nodes that are located farthest from each other, and each of the two middle end nodes is connected to a corresponding side of a supporting bar by a guiding bar; the two guiding bars do not intersect each other, and the junction between each guiding bar and the supporting bar is substantially located at a middle portion in the axial direction of the flared section.

A section of the supporting net frame is a transition section, a ratio of an axial length of the transition section before being compressed to an axial length of the transition section after being compressed is 1. Since the axial length of the transition section keeps constant before and after being compressed, compared with the rbombic grids generally used in the prior art, the change of the axial length of the supporting net frame before and after being compressed can be reduced.

Preferably, the axial length of the transition section equals at least 40% of a total length of the supporting net frame. Only if a ratio of the axial length of the transition section to the total length of the supporting net frame reaches 40% or more, the effect of the transition section can appear, that is, due to the transition section, a length of a compressed supporting net frame can be reduced so as to meet the requirement of adaptability for bending, such that the supporting net frame can easily reach a predetermined site inside a human body, thereby ensuring that an operation is preformed favorably.

Although the transition section can reduce the change of the axial length of the supporting net frame before and after being compressed, it is not true that the longer the transition section the better. The reason is that, although the axial lengths of the rhombic grids generate great changes before and after being compressed, the structures of the rhombic grids contribute to the strength of the supporting net frame, and resists the impact of blood flows for a long time, and the structures of the rhombic grids enable the supporting net frame to be compressed and positioned inside the sheath pipe.

Therefore, preferably, the axial length of the transition section equals 40%-90% of the total length of the supporting net frame. Further preferably, the axial length of the transition section equals 50%-80% of the total length of the supporting net frame.

As the simplest embodiment, the transition section is formed by a plurality of straight rods extending along the axial direction of the valve stent, and the straight rods are evenly distributed along the circumference of the valve stent.

An end portion of each straight rod of the transition section is connected with a corresponding rhombic vertice, and particularly, the end of each straight rod of the transition section is connected with a vertice of a rhombic grid facing the transition section.

The valve stent provided by the present invention is easy to realize, with a production technologies and production efficiency substantially unaffected, it is only required to modify the design drawings of the valve stent and processing laser cutting of the valve stent base on the modified drawings, the manufacturing cost will not increase, all subsequent processing technologies and moulds can use the existing ones, and thus the cost for the improvement is low.

The present invention further provides a valve replacement device, which includes the aforementioned valve stent and a prosthesis valve fixed inside the supporting net frame.

Both the supporting net frame and the inflow portion of the valve stent are covered by films; the prosthesis valve can be sutured onto an inner wall of the valve stent, and can also be mounted and fixed by other known means.

The present invention improves the structures of existing memory alloy self-expandable valve stents, eliminates the vertices existing in isolation and present at non-end portions of the valve stents, preventing spines from appearing after the valve stent is compressed while maintaining the mechanical properties of the original structural, and thereby resolving the problem of sheath damage caused by the spines.

### BRIEF DESCRIPTION OF THE DRAWINGS

Fig. 1 is a schematic view of a valve stent in the prior art;
Fig. 2 is a schematic view showing that an isolated end node pierces a sheath, according to the prior art;
Fig. 3 is a structural schematic view of a valve stent of the present invention (wherein the back side thereof is omitted);
Fig. 4 is a perspective view of the valve stent of the present invention;
Fig. 5 is a structural schematic view of a valve stent according to a second embodiment of the present invention (wherein the back side thereof is omitted).

In the drawings, the corresponding relationship between numbers and components are as follows:
1, outflow portion; 2, second grid portion; 3, transition section; 4, first grid portion; 5, inflow portion; 6, supporting bar; 7, straight rod; 8, guiding bar; 9, end node; 10, end node; 11, sheath; 12, supporting net frame.

### DETAILED DESCRIPTION OF THE PREFERRED EMBODIMENT

The present invention will be further described hereafter with reference to the accompany drawings, taking a pulmonary artery stent as an example.

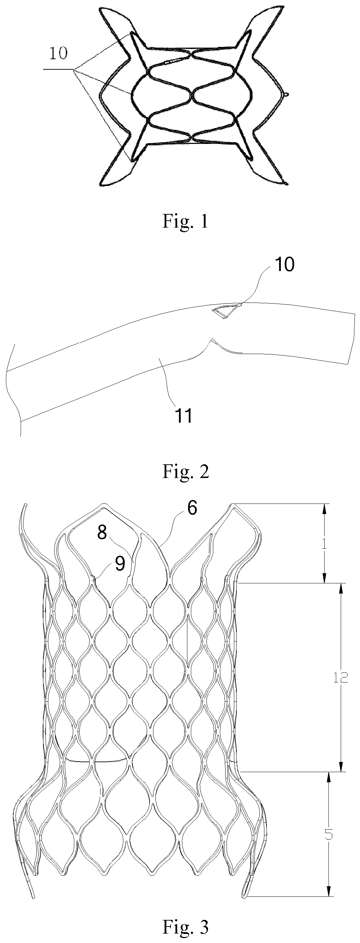

There are end nodes existing in isolation in pulmonary artery stents in the prior art. For example, there are end nodes 10 in a pulmonary artery stent shown in Fig. 1. The end nodes 10 are not located at any end portion of the pulmonary artery stent, after the pulmonary artery stent is compressed into a sheath, the isolated end nodes 10 are prone to be deformed into spines and pierce the sheath 11 during the retracting process of the sheath 11, as shown in Fig. 2.

### Embodiment I

As shown in Fig. 3, the present invention provides a pulmonary artery stent which can be used safely, including a supporting net frame 12, and an inflow portion 5 and an outflow portion 1 that are connected to two axial ends of the supporting net frame 12, respectively.

The inflow portion 5 and the outflow portion 1 expand radially outwardly to form flared sections. When the pulmonary artery stent is released inside a human body, the outflow portion 1 of the pulmonary artery stent is a precedent release end, and the outflow portion 1 is connected to all end nodes 9 at a corresponding side of the supporting net frame 12. A back side of the valve stent is omitted in Fig. 3, and only a front side of the valve stent is shown.

An outer rim of the outflow portion 1 is formed by a plurality of curved supporting bars 6. Every four adjacent end nodes 9 are assigned as a group, which are connected together by a supporting bar 6 and two guiding bars 8. Two ends of the supporting bar 6 are respectively connected with two end nodes 9 that are the located farthest from each other, each of the two middle end nodes 9 is connected to a corresponding side of the supporting bar 6 by a respective guiding bar 8. The two guiding bars 8 do not intersect each other, and the junction between each guiding bar 8 and the supporting bar 6 is substantially located at an axial middle portion of the outflow portion 1.

Each guiding bar 8 gradually deviates away from the supporting net frame 12 from the corresponding end node 9 to the supporting bar 6. an acute angle is formed between each guiding bar 8 and the supporting bar 6 at the junction. An included angle between a line connecting two ends of a respective guiding bar 8 and an axis of the valve stent is 30 degrees.

### Embodiment II

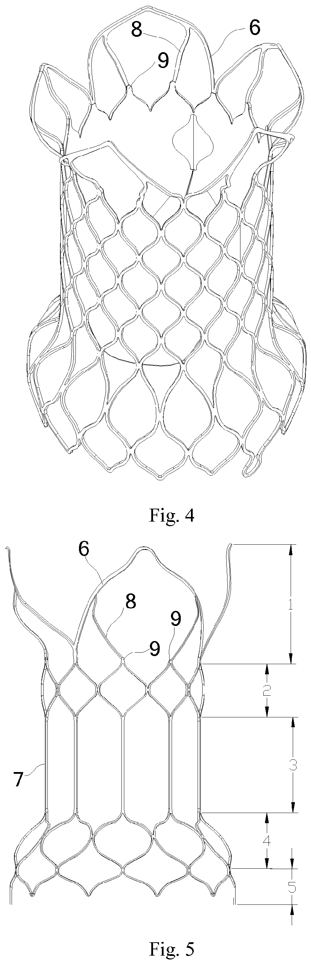

As shown in Fig. 5, the present invention provides another pulmonary artery stent which can be used safely, including a supporting net frame, an inflow portion 5 and an outflow portion 1 connected to two axial ends of the supporting net frame respectively. A middle portion of the supporting net frame is a transition section 3.

One end of the transition section 3 is connected with the inflow portion 5 via a first grid portion 4, and the other end of the transition section 3 is connected with the outflow portion 1 via a second grid portion 2. Both the first grid portion 4 and the second grid portion 2 are formed by continuous rhombic structures. Compared with the transition section 3, the first grid portion 4 expands radially to form a flared section. Rims of each rhombic structure are not exact straight lines but curved outwards slightly, and the number of the rhombic structures of the first grid portion 4 equals the number of the rhombic structures of the second grid portion 2.

The transition section 3 is formed by a plurality of straight rods 7 extending along an axial direction of the valve stent. The straight rods 7 are evenly distributed along a circumference of the valve stent, and an axial length of the transition section is about 45% of a total length of the supporting net frame.

Ends of each straight rod 7 of the transition section 3 are connected with a corresponding rhombic vertice of a rhombic unit, which faces, i.e., located adjacent to, the transition section 3. It can be seen from Fig. 5 that the straight rods 7 of the transition section 3 and the rims of the adjacent rhombic units cooperatively form hexagons, and interior angles of the hexagons are all obtuse angles.

The present invention adopts a design structure of a self-expandable valve stent, which realizes the retraction of the valve stent by the deformation of the rhombic grid section, such that the valve stent can be compressed into a sheath. Moreover, the valve stent can generate a uniform radial supporting force after being implanted into a human body, and thus the valve can be prevented from moving and dropping. As the isolated end nodes are connected to the supporting bars by the guiding bars, the risk that the end nodes pierce the sheath is eliminated, without changing adaptability of the flared section to the blood vessel.

Furthermore, the embodiment II of the present invention can reduce almost 50% of the amount of metal material used by the valve stent (which is usually memory alloy, the present invention uses nickel-titanium alloy), not only reduces a diameter of the valve stent after being compressed, but also improves the adaptability for bending, so that the passing-through performance of the valve in the blood vessel is further enhanced.

The present invention further provides a valve replacement device, which includes an aforementioned valve stent and a prosthesis valve fixed inside the supporting net frame. When the valve stent reaches a predetermined site inside a human body via a transport system, the valve stent is released from a sheath and then expands, and the prosthesis valve fixed inside the valve stent replaces the natural valve of the human body to realize the function of enabling blood to pass unidirectionally.

## Claims

1. A valve stent, comprising:
a tubular supporting net frame (12), and
a flared section (1) connected to a corresponding end of the supporting net frame (12);
wherein the flared section (1) is connected to all end nodes (9) located at a corresponding side of the supporting net frame (12)
**characterized in that**
every four adjacent end nodes are assigned as group, wherein two ends of a supporting bar (6) are respectively connected with two end nodes (9) that are located farthest from each other,
and each of the two middle end nodes (9) is connected to a corresponding side of the supporting bar (6) by a guiding bar (8).

2. The valve stent according to claim 1, wherein the flared section (1)
is located at a precedent release end of the valve stent.

3. The valve stent according to claim 1 or 2, wherein an outer rim
of the flared section is formed by a plurality of curved supporting bars (6), and the end nodes (9) of the supporting net frame (12) corresponding to the positions of the supporting bars (6) are all connected to the supporting bars (6).

4. The valve stent according to claim 3, wherein the end nodes (9)
intersect the supporting bars (6) or are connected with the supporting bars (6) tangentially and intersectingly by guiding bars (8).

5. The valve stent according to claim 4, wherein each of the
guiding bars (8) gradually deviates away from the supporting net frame (12) along an extending path from a corresponding end node (9) to a corresponding supporting bar (6).

6. The valve stent according to claim 5, wherein an included
angle formed by a line connecting two ends of the extending path and an axis of the valve stent is in a range of 0-70 degrees.

7. The valve stent according to claim 5, wherein an included angle
formed by a line connecting two ends of the extending path and an axis of the valve stent is in a range of 20-60 degrees.

8. The valve stent according to claim 6, wherein, an intersection
angle formed between the guiding bar (8) and the supporting bar (6) at the junction is an acute angle.

9. The valve stent according to claim 1 or 2, wherein a section of
the supporting net frame (12) is a transition section, and a ratio of an axial length of the transition section before being compressed to an axial length of the transition section after being compressed is 1.

10. The valve stent according to any of the preceding claims, wherein the junction between each guiding bar (8) and a supporting bar (6) is located at a middle portion in the axial direction of the flared section (1).

11. A valve replacement device, comprising:
a valve stent of any one of claims 1-10; and
a prosthesis valve fixed inside the supporting net frame (12).

## Patentansprüche

1. Ein Klappenstent mit:
einer schlauchförmigen stützenden Netzstruktur (12) und
einem aufgeweiteten Bereich (1), der mit einem entsprechenden Ende der stützenden Netzstruktur (12) verbunden ist,
wobei der aufgeweitete Bereich (1) mit allen Endknoten (9) an einer entsprechenden Seite der stützenden Netzstruktur (12) verbunden ist,
**dadurch gekennzeichnet, dass**
jeweils vier aneinanderliegenden Endknoten als Gruppe gelten, wobei zwei Enden einer Trägerstange (6) jeweils mit den beiden Endknoten (9) verbunden sind, die am weitesten auseinander liegen, und die beiden mittleren Endknoten (9) jeweils über eine Führungsstange (8) mit einer entsprechenden Seite der Trägerstange (6) verbunden sind.

2. Der Klappenstent gemäß Anspruch 1, wobei der aufgeweitete Bereich (1) sich an einem vorstehenden Auslöseende des Klappenstents befindet.

3. Der Klappenstent gemäß Anspruch 1 oder 2, wobei ein äußerer Rand des aufgeweiteten Bereichs von einer Mehrzahl gekrümmter Trägerstangen (6) gebildet wird und die Endknoten (9) der stützenden Netzstruktur (12), die den Positionen der Trägerstangen (6) entsprechen, alle mit den Trägerstangen (6) verbunden sind.

4. Der Klappenstent gemäß Anspruch 3, wobei die Endknoten (9) die Trägerstangen (6) kreuzen oder tangential und kreuzend über Führungsstangen (8) mit den Trägerstangen (6) verbunden sind.

5. Der Klappenstent gemäß Anspruch 4, wobei jede Führungsstange (8) graduell entlang eines Pfads von der stützenden Netzstruktur (12) abweicht, der sich vom entsprechenden Endknoten (9) bis zu einer entsprechenden Trägerstange (6) erstreckt.

6. Der Klappenstent gemäß Anspruch 5, wobei ein eingeschlossener Winkel, der von einer Linie gebildet wird, die zwei Enden des Ausdehnungspfads und eine Achse der Klappenstents verbindet, zwischen 0 und 70 Grad liegt.

7. Der Klappenstent gemäß Anspruch 5, wobei ein eingeschlossener Winkel, der von einer Linie gebildet wird, die zwei Enden des Ausdehnungspfads und eine Achse der Klappenstents verbindet, zwischen 20 und 60 Grad liegt.

8. Der Klappenstent gemäß Anspruch 6, wobei ein Schnittwinkel zwischen Führungsstange (8) und Trägerstange (6) einem spitzen Winkel entspricht.

9. Der Klappenstent gemäß Anspruch 1 oder 2, wobei ein Abschnitt der stützenden Netzstruktur (12) einen Übergangsbereich bildet und ein Verhältnis zwischen axialer Länge des Übergangsbereichs vor der Kompression und axialer Länge des Übergangsbereichs nach der Kompression 1 beträgt.

10. Der Klappenstent gemäß einem der vorstehenden Ansprüche, wobei der Schnittpunkt zwischen einer Führungsstange (8) und einer Trägerstange (6) in einem mittleren Bereich in axialer Richtung des aufgeweiteten Bereichs (1) liegt.

11. Eine Klappenersatzvorrichtung mit:
einem Klappenstent gemäß einem der Ansprüche 1 bis 10 und
einer innerhalb der stützenden Netzstruktur (12) befestigten Klappenprothese.

## Revendications

1. Stent de valvule, comprenant :
une ossature de treillis de support tubulaire (12), et
une section évasée (1) reliée à une extrémité correspondante de l'ossature de treillis de support (12) ;
où la section évasée (1) est reliée à tous les nœuds d'extrémité (9) situés au niveau d'un côté correspondant de l'ossature de treillis de support (12)
**caractérisé en ce que**
chaque série de quatre nœuds d'extrémité successifs forme un groupe, dont les deux nœuds d'extrémité (9) les plus éloignés l'un de l'autre sont respectivement reliés à deux extrémités d'une barre de support (6), et dont chacun des deux nœuds d'extrémité centraux (9) est relié à un côté correspondant de la barre de support (6) par une barre de guidage (8).

2. Stent de valvule selon la revendication 1, dans lequel la section évasée (1) est située à une extrémité de libération antérieure du stent de valvule.

3. Stent de valvule selon la revendication 1 ou 2, dans lequel un bord extérieur de la section évasée est formé par une pluralité de barres de support incurvées (6), et les nœuds d'extrémité (9) de l'ossature de treillis de support (12) correspondant aux positions des barres de support (6) sont tous reliés aux barres de support (6).

4. Stent de valvule selon la revendication 3, dans lequel les nœuds d'extrémité (9) croisent les barres de support (6) ou sont reliés aux barres de support (6) de manière tangentielle et de manière à les croiser par les barres de guidage (8).

5. Stent de valvule selon la revendication 4, dans lequel chacune des barres de guidage (8) s'écartent progressivement de l'ossature de treillis de support (12) le long d'une trajectoire d'étendue allant d'un nœud d'extrémité correspondant (9) vers une barre de support correspondante (6).

6. Stent de valvule selon la revendication 5, dans lequel un angle intérieur formé par une droite reliant deux extrémités de la trajectoire d'étendue et un axe du stent de valvule se situe dans une plage allant de 0 à 70 degrés.

7. Stent de valvule selon la revendication 5, dans lequel un angle intérieur formé par une droite reliant deux extrémités de la trajectoire d'étendue et un axe du stent de valvule se situe dans une plage allant de 20 à 60 degrés.

8. Stent de valvule selon la revendication 6, dans lequel un angle d'intersection formé à la jonction entre la barre de guidage (8) et la barre de support (6) est un angle aigu.

9. Stent de valvule selon la revendication 1 ou 2, dans lequel une section de l'ossature de treillis de support (12) est une section de transition, et un rapport d'une longueur axiale de la section de transition avant d'être comprimée sur une longueur axiale de la section de transition après qu'elle soit comprimée est de 1.

10. Stent de valvule selon l'une quelconque des revendications précédentes, dans lequel la jonction entre chaque barre de guidage (8) et une barre de support (6) est située au niveau d'une partie centrale dans la direction axiale de la section évasée (1).

11. Dispositif de remplacement de valvule, comprenant :
un stent de valvule selon l'une quelconque des revendications 1 à 10 ; et
une valvule prothétique fixée à l'intérieur de l'ossature de treillis de support (12).
